# EUROPEAN PATENT APPLICATION

(11) **EP 2 157 665 A1**
(43) Date of publication of application: **24.02.2010**
(21) Application number: 09158876.4
(22) Date of filing: 27.04.2009
(51) Int. Cl.: H01Q 9/04, H01Q 13/10

(54) **UWB antenna and detection apparatus for transportation means**

(30) Priority: 22.08.2008 TW 97132026
(71) Applicant: Industrial Technology Research Institute, Hsinchu 31040 (TW)
(72) Inventor: Tao, Teh Ho, Hsinchu City, Taiwan R.O.C. (TW); Vlasov, Denis Vladimirovich, Hsinchu City 300 (TW); Khripkov, Alexander Nikolayevich, Hsinchu City 300 (TW); Chang, Kuang I, Tucheng City, Taipei County 236 (TW); Chow, Zu Sho, Jhubei City, Hsinchu County (TW); Chen, Cheng Foo, Jhongjheng District, Taipei City 100 (TW); Chang, Tzu Ming, Taoyuan City, Taoyuan County 330 (TW); Yang, Ching Huan, Da-an District, Taipei City 106 (TW)
(74) Representative: 2K Patentanwälte Blasberg Kewitz & Reichel

(57) **Abstract**

A UWB antenna for transportation means comprises a metallic screen, a dielectric substrate and a rectangular printed metallic patch. The dielectric substrate is disposed on the printed metallic patch. The printed metallic patch is disposed on the dielectric substrate, and has a horizontal trench gap and two vertical trench gaps. The horizontal trench gap is parallel to the long side of the rectangular printed metallic patch, and the vertical trench gaps respectively extend upward from each end of the horizontal trench gap to form two resonance contours, i.e. the trench gaps for a U-shaped slot.

## Description

### 1. Technical Field

The present invention relates to an antenna, and more particularly, to an ultra-wide band (UWB) antenna and detection apparatus for transportation means.

### 2. Background

Traffic accidents are one of the most frequent causes of death. Therefore, road safety has been the public focus and a top priority when one purchases a car. As statistics show, most car accidents can be attributed to human factors, such as over speeding, driving while drunk, falling asleep while driving, or even suffering a heart attack. Unfortunately, most drivers drive alone and when danger comes, e.g. a heart attack or falling asleep while driving, the driver is often not aware of the danger or has too little time to deal with the emergency at hand.

Accordingly, monitoring a driver's physiological status to warn the driver of abnormity may be a solution to the aforesaid issue. U.S. patent publication No. 2007/0055164, "Physiological Status Monitoring System And Method" discloses a physiological status monitoring method and system utilizing a biosensor and a CPU to monitor physiological signals.

U.S. patent No. 6,462,701, "System And Method For Controlling Air Bag Deployment Systems" discloses a method and system for controlling air bag deployment systems based on the presence, position, size and weight of a person in an automobile. The system, comprising a radar transceiver and a CPU, can control the deployment of an air bag system by detecting a driver's physiological parameters according to the radar signals transmitted to and received from the driver.

In view of the aforesaid conventional techniques, extensive research has been conducted on the design of transceivers installed in automobiles to monitor drivers' physiological status or parameters. However, none of the aforesaid conventional techniques focuses on the structure of antenna. Accordingly, there is a need to design an antenna which exhibits the features of low power, small size and high measurement accuracy, for transportation means, so as to meet industrial requirements.

### SUMMARY

The UWB antenna according to some embodiments is installed in a driver's seat or a dashboard to detect a driver's respiratory and heartbeat signals without contacting the driver.

The UWB antenna for transportation means according to embodiments of the present invention comprises a metallic screen, a dielectric substrate and a rectangular printed metallic patch. The dielectric substrate is disposed on the metallic screen. The rectangular printed metallic patch is disposed on the dielectric substrate with a horizontal trench gap and two vertical trench gaps thereon. The horizontal gauging is substantially parallel to the long side of the metallic patch. The vertical trench gaps respectively extend upward from each end of the horizontal trench gap to form two resonance contours.

The UWB-based detecting apparatus for transportation means according to embodiments of the present invention comprises a UWB antenna and a receiver. The UWB antenna is configured to emit UWB signals. The receiver is configured to receive the UWB signals reflected from a body such as human or life being.

### BRIEF DESCRIPTION OF THE DRAWINGS

The objectives and advantages of the present invention will become apparent upon reading the following description and upon referring to the accompanying drawings, of which:
FIG. 1 shows a schematic view of a UWB antenna for transportation means according to some exemplary embodiments;
FIG. 2 shows a schematic view of a UWB antenna according to some exemplary embodiments emitting a UWB signal toward a human body;
FIG. 3A shows the relationship between the frequency and the gain of a UWB antenna according to some exemplary embodiments;
FIG. 3B shows the relationship between the frequency and the voltage standing wave ratio of a UWB antenna according to some exemplary embodiments;
FIG. 3C shows radiation patterns of a UWB antenna according to some exemplary embodiments corresponding to different frequencies;
FIG. 4 shows a schematic view of a UWB-based detecting apparatus for transportation means according to some exemplary embodiments; and
FIG. 5 shows a schematic view of another UWB-based detecting apparatus for transportation means according to some exemplary embodiments.

### DETAILED DESCRIPTION

Exemplary Embodiments will now be described more fully with reference to the accompanying drawings. The present invention may, however, be embodied in many different forms and should not be construed as limited to the embodiments set forth herein. Rather, these embodiments are provided so that this disclosure will be thorough and complete, and will fully convey the scope of the present invention to those skilled in the art.

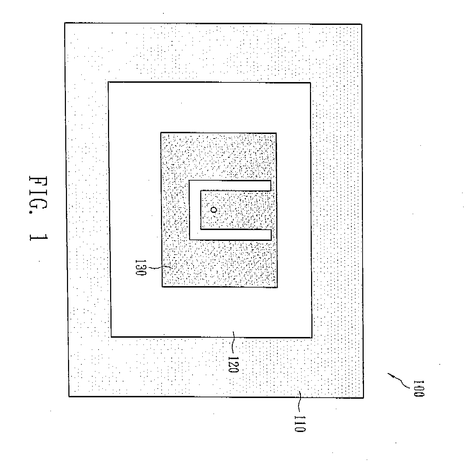

FIG. 1 shows a schematic view of a UWB antenna for transportation means according to some exemplary embodiments. The UWB antenna 100 is installed in a driver's seat or a dashboard for emitting a UWB signal, and comprises a metallic screen 110, a dielectric substrate 120 and a printed metallic patch 130, wherein the metallic screen 110, the dielectric substrate 120 and the printed metallic patch 130 are all rectangular. The area of the metallic screen 110 is larger than that of the dielectric substrate 120. The area of the dielectric substrate 120 is larger than that of the printed metallic patch 130. The dielectric substrate 120 is disposed on the metallic screen 110. The printed metallic patch 130 is disposed on the dielectric substrate 120. The printed metallic patch 130 comprises a horizontal trench gap and two vertical trench gaps, wherein the horizontal trench gap is substantially parallel to the long side of the printed metallic patch 130, and the vertical trench gaps respectively extend upward from each end of the horizontal trench gap to form two resonance contours. The structural parameters of the UWB antenna 100 can be adjusted to achieve the same resonance frequency for the two resonance contours.

As shown in FIG. 1, the horizontal length of the metallic screen 110 is between 50 and 70 millimeters. The vertical height of the metallic screen 110 is between 45 and 65 millimeters. The horizontal length of the dielectric substrate 120 is between 40 and 60 millimeters. The vertical height of the dielectric substrate 120 is between 35 and 55 millimeters. The widths of the vertical trench gaps and the horizontal trench gap are substantially the same. That is, the widths of the vertical trench gaps and the horizontal trench gap are between 1.5 and 2 millimeters. The length of the horizontal trench gap is between 10 and 14 millimeters. The lengths of the vertical trench gaps are between 8 and 12 millimeters.

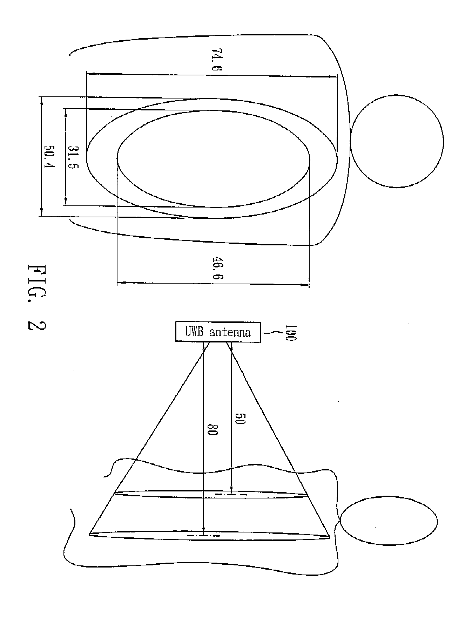

FIG. 2 shows a schematic view of the UWB antenna 100 emitting a UWB signal toward a human body. The electronic wave emitted from the UWB antenna 100 covers an ellipse-shaped area, wherein the size of the ellipse-shaped area is in direct ratio to the distance between the UWB antenna 100 and the human body. As shown in FIG. 2, if the distance between the UWB antenna 100 and the human body is 80 centimeters, then the major axis of the ellipse is 74.6 centimeters, and the minor axis of the ellipse is 50.4 centimeters. If the distance between the UWB antenna 100 and the human body is 50 centimeters, then the major axis of the ellipse is 46.6 centimeters, and the minor axis of the ellipse is 31.5 centimeters. The emitted UWB signal exhibits vertical angle and a horizontal angle, wherein the horizontal angle is between 40 and 70 degrees, the vertical angle is between 50 and 80 degrees, and the horizontal angle exhibits an angular bias of between 1 and 10 degrees relative to a vertical axis. Preferably, the horizontal angle is between 50 and 60 degrees, the vertical angle is between 60 and 70 degrees, and the horizontal angle exhibits an angular bias of between 2 and 5 degrees relative to a vertical axis.

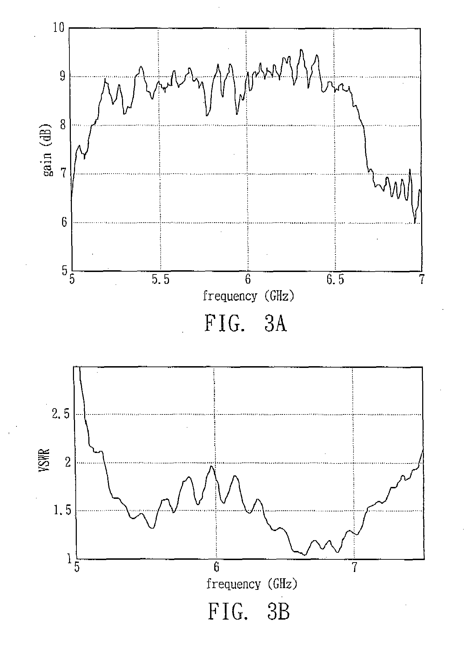

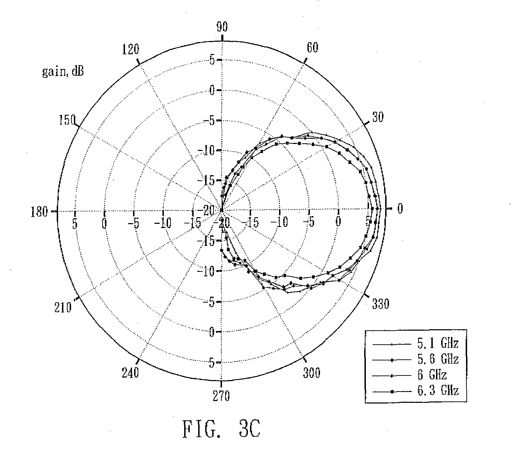

FIG. 3A shows the relationship between the frequency and the gain of the UWB antenna 100. FIG. 3B shows the relationship between the frequency and the voltage standing wave ratio (VSWR) of the UWB antenna 100. The optimum operating frequency of the UWB antenna 100 is between 5.2 and 7.4 GHz. Accordingly, the fractional bandwidth of the UWB antenna 100 is about 35%. As shown in FIG. 3A, the gain corresponding to most frequencies between 5 and 7 GHz is greater than 8dB. Likewise, as shown in FIG. 3B, the VSWR corresponding to most frequencies between 5 and 7 GHz is lesser than 2. Therefore, as shown in FIG. 3A and 3B, the UWB signal emitted by the UWB antenna 100 when operating in its optimum operating frequency is of relatively high quality. FIG. 3C shows radiation patterns of the UWB antenna 100 corresponding to different frequencies. As shown in FIG. 3C, the beamwidth of the UWB antenna 100 is relatively wide, and the angles of the beamwidth in both the E-plane and H-plane are between 60 and 70 degrees.

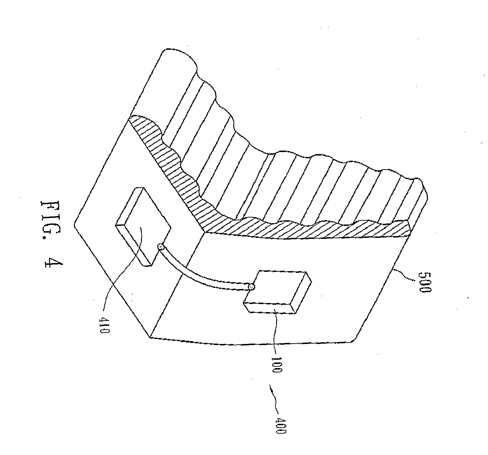

FIG. 4 shows a schematic view of a UWB-based detecting apparatus for transportation means according to some exemplary embodiments. The UWB-based detecting apparatus 400 comprises the UWB antenna 100 and a transceiver 410. The UWB-based detecting apparatus 400 is installed at the back of a driver's seat 500, and the transceiver 410 is installed under the driver's seat 500. The driver's position is within the range covered by the UWB signal emitted from the UWB antenna 100 and UWB antenna 100 also receives UWB signal reflected from the driver. The transceiver 410 analyzes the UWB signal from the UWB antenna 100. The driver's physiological status is determined based on the phase difference of the detecting electronic wave reflected from the driver's organs and an electronic wave.

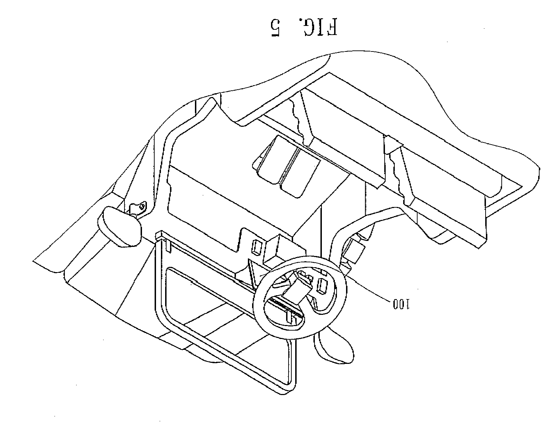

FIG. 5 shows a schematic view of another UWB-based detecting apparatus for transportation means according to some exemplary embodiments, wherein the UWB antenna 100 is installed in a dashboard.

The UWB antenna for transportation means according to some embodiments emits UWB electronic waves. Therefore, by applying the UWB antenna according to some embodiments, the frequency density of the dispersed power is lowered, which not only alleviates the destructive effect upon human bodies caused by dispersed electronic waves, but also reduces the interference between the emitted UWB electronic waves and the electronic waves emitted from other electronic devices to a negligible degree. In addition, from a practical point of view, the application of UWB electronic waves reduces power consumption, and the manufacturing cost is lowered due to the smaller size of the UWB antenna. Furthermore, the cut plane of the UWB electronic waves emitted from the UWB antenna according to some embodiments exhibits an elliptical shape, which can easily cover a driver's body, including the heart area of the driver. Therefore, measurement accuracy of the UWB antenna according to some embodiments is relatively high.

The above-described exemplary embodiments are intended to be illustrative only. Those skilled in the art may devise numerous alternative embodiments without departing from the scope of the following claims.

## Claims

1. An ultra-wide band (UWB) antenna for transportation means for emitting a UWB signal, comprising:
a metallic screen;
a dielectric substrate disposed on the metallic screen; and
a rectangular printed metallic patch disposed on the dielectric substrate with a horizontal trench gap and two vertical trench gaps thereon;
wherein the horizontal trench gap is substantially parallel to a long side of the rectangular printed metallic patch, and the vertical trench gaps respectively extend upward from two ends of the horizontal trench gap to form two resonance contours.

2. The UWB antenna of Claim 1, wherein both the resonance contours exhibit substantially the same resonance frequency.

3. The UWB antenna of Claim 1, wherein the widths of the vertical trench gaps and the horizontal trench gap are substantially the same.
and/ or
wherein the widths of the vertical trench gaps and the horizontal trench gap are between 1.5 and 2 millimeters.

4. The UWB antenna of Claim 1, wherein the length of the horizontal trench gap is between 10 and 14 millimeters.
and/ or
wherein the lengths of the vertical trench gaps are between 8 and 12 millimeters.

5. The UWB antenna of Claim 1, wherein the metallic screen and the dielectric substrate are both rectangular.

6. The UWB antenna of Claim 1, wherein the area of the metallic screen is larger than that of the dielectric substrate, and the area of the dielectric substrate is larger than that of the printed metallic patch.

7. The UWB antenna of Claim 5, wherein the horizontal length of the dielectric substrate is between 40 and 60 millimeters and/or
wherein the vertical height of the dielectric substrate is between 35 and 55 millimeters.

8. The UWB antenna of Claim 5, wherein the horizontal length of the metallic screen is between 50 and 70 millimeters and/or
wherein the vertical height of the metallic screen is between 45 and 65 millimeters.

9. The UWB antenna of Claim 1, wherein the horizontal angle of the emitted signal is between 40 and 70 degrees, the vertical angle of the emitted signal is between 50 and 80 degrees, and the horizontal angle exhibits an angular bias of between 1 and 10 degrees relative to a vertical axis.

10. The UWB antenna of Claim 9, wherein the horizontal angle is between 50 and 60 degrees.
and/or
wherein the vertical angle is between 60 and 70 degrees.

11. The UWB antenna of Claim 9, wherein the horizontal angle exhibits an angular bias of between 2 and 5 degrees relative to a vertical axis.

12. The UWB antenna of Claim 9, which exhibits an optimum operating frequency of between 5.2 and 7.4 GHz.

13. The UWB antenna of Claim 1, which is installed in a driver's seat or a dashboard.

14. An ultra-wide band (UWB)-based detecting apparatus for transportation means, comprising:
a UWB antenna according to Claim 1; and
a receiver;
wherein the UWB antenna is configured to emit and receive UWB signals, and the transceiver is configured to transmit and receive UWB signals from the antenna and to process the UWB signals reflected from a driver of the transportation means.

15. The detecting apparatus of Claim 14, wherein the UWB antenna is installed at the back of a driver's seat, and the transceiver is installed under the driver's seat.
